# EUROPEAN PATENT APPLICATION

(11) **EP 4 353 821 A1**
(43) Date of publication of application: **17.04.2024**
(21) Application number: 22200543.1
(22) Date of filing: 10.10.2022
(51) Int. Cl.: C12N 9/12, A61K 38/16, A61K 38/45, C12N 15/90, C12N 9/22

(54) **TRANSPOSASE MUTANT WITH ENHANCED EFFICIENCY OF GENE TRANSFER**

(71) Applicant: Max-Delbrück-Centrum für Molekulare Medizin in der Helmholtz-Gemeinschaft, 13125 Berlin (DE)
(72) Inventor: IVICS, Zoltan, 13125 Berin (DE); OCHMANN, Matthias, 63225 Langen (DE)
(74) Representative: Moré, Solveig Helga

(57) **Abstract**

The invention relates to the field of transposases. In particular, it provides a polypeptide comprising or consisting of a Tc1/*mariner* superfamily transposase, e.g., a Sleeping Beauty transposase, having a substitution in amino acid position 124. The transposases of the invention were found to have an enhanced transposition efficiency. The invention also provides nucleic acids encoding said transposases, cell expressing it, kits and pharmaceutical compositions comprising said transposase, e.g., for use in gene therapy, e.g., adoptive T cell therapy, or uses and methods for gene delivery into the genome of a cell.

## Description

The invention relates to the field of transposases. In particular, it provides a polypeptide comprising or consisting of a Tc1/*mariner* superfamily transposase, e.g., a Sleeping Beauty transposase, having a substitution in amino acid position 124. The transposases of the invention were found to have an enhanced transposition efficiency. The invention also provides nucleic acids encoding said transposases, cell expressing it, kits and pharmaceutical compositions comprising said transposase, e.g., for use in gene therapy, e.g., adoptive T cell therapy, or uses and methods for gene delivery into the genome of a cell.

The capability of nucleic acids to integrate their sequence into a new locus evolved in manifold ways. The large family of transposons first described by Barbara McClintock in maize [1] have the ability to move their genetic information within the genome. Transposable elements (TEs) can be classified into two groups. Class I TE are the so called retrotransposons that follow a copy-and-paste mechanism and use an RNA intermediate for this process [2]. Class II transposons rely solely on DNA intermediates for their transposition process. In this category subclass I transposons follow a cut-and-paste mechanism, during which the transposon is excised from one genomic location and reintegrates at a different location [2]. The Tc1/*mariner* superfamily follows this canonical cut-and-paste mechanism. The TEs of this superfamily are flanked by terminal inverted repeats (TIRs) and harbor a gene encoding a transposase, which is an enzymatic factor that catalyzes the transposition reaction [2]. The transposase binds to the TIRs, excises the TE from the donor locus and reintegrates it adjacent to a TA target sequence, which ultimately leads to a TA target site duplication [2].

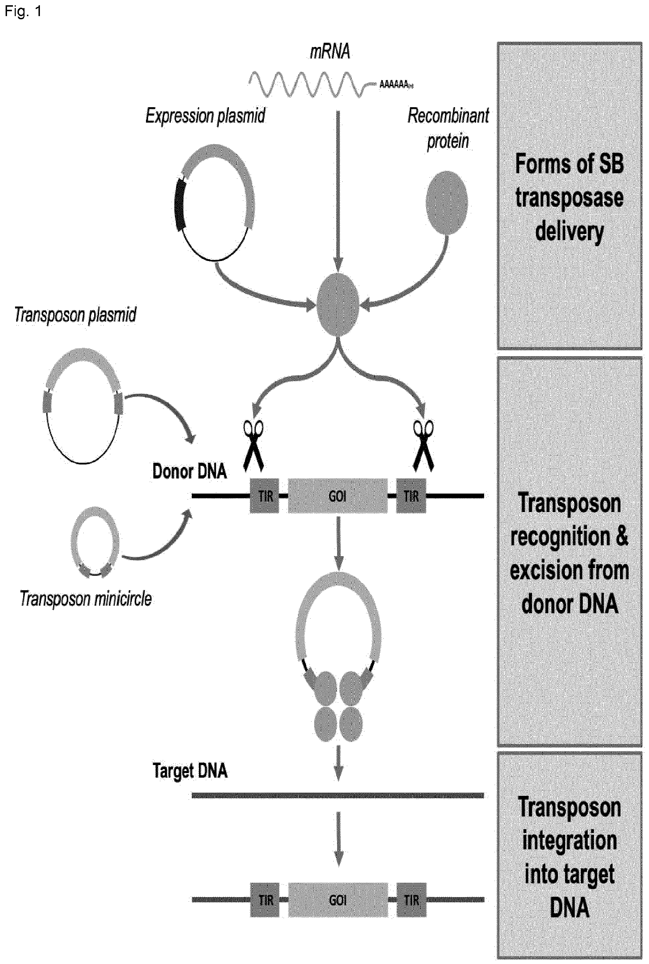

The minimally required components for the transposition reaction are the TIRs and transposase. TEs that contain both of these components are therefore considered as autonomous TEs [3]. Many autonomous TEs have given rise to non-autonomous derivates by modifications in the transposase coding region. These non-autonomous TEs can still be mobilized, but need a functional transposase expressed by another element in the same cell [3]. This *trans*-complementarity between two functional components (the transposase and the specific TIRs that are recognized and mobilized by the transposase) are the basis of turning transposons into genetic vector systems suitable to move any gene of interest in the genome of a host cell **(****Fig. 1****).**

A member of the Tc1/*mariner* superfamily is the *Sleeping Beauty* (SB) transposon [3]. It was reconstructed from fossil DNA sequences within fish genomes and was the first DNA transposon shown to be active in vertebrates [3]. It is widely used as a genetic engineering tool in various preclinical studies and clinical trials [4]. The structural and biochemical features of the SB transposase catalyzing the transposition reaction are of special interest, because based on the enzymatic activity of the transposase the efficiency of SB transposon integration into target cell genomes can be enhanced. The SB transposase is composed of an N-terminal DNA binding domain (amino acids (aa 1-110) and a C-terminal catalytic domain (DDE) (aa 114-340) [3]. Both domains are connected by a flexible linker region that harbors a nuclear localization signal (NLS) (aa 97-123) [3]. The DNA binding domain consists of two subdomains, the PAI and RED subdomain, each of them forming a helix-turn-helix (HTH) motif important for the recognition and binding of transposon DNA [3,5]. The catalytic domain, with its three conserved amino acids in the catalytic center (D153, D244, E279 (DDE)), catalyzes DNA hydrolysis reactions, which are required for excision and transesterification taking place in the integration reaction [5-7].

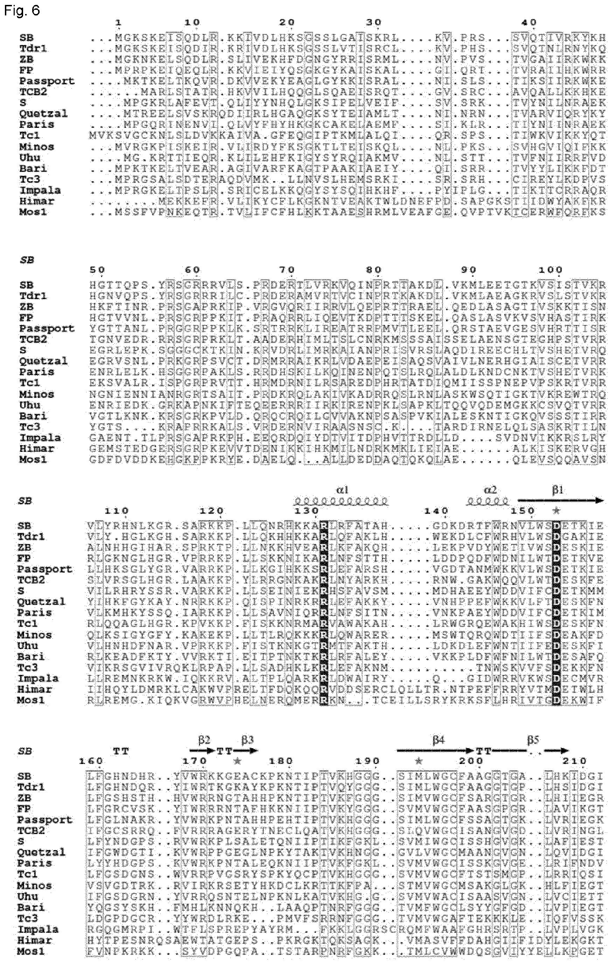

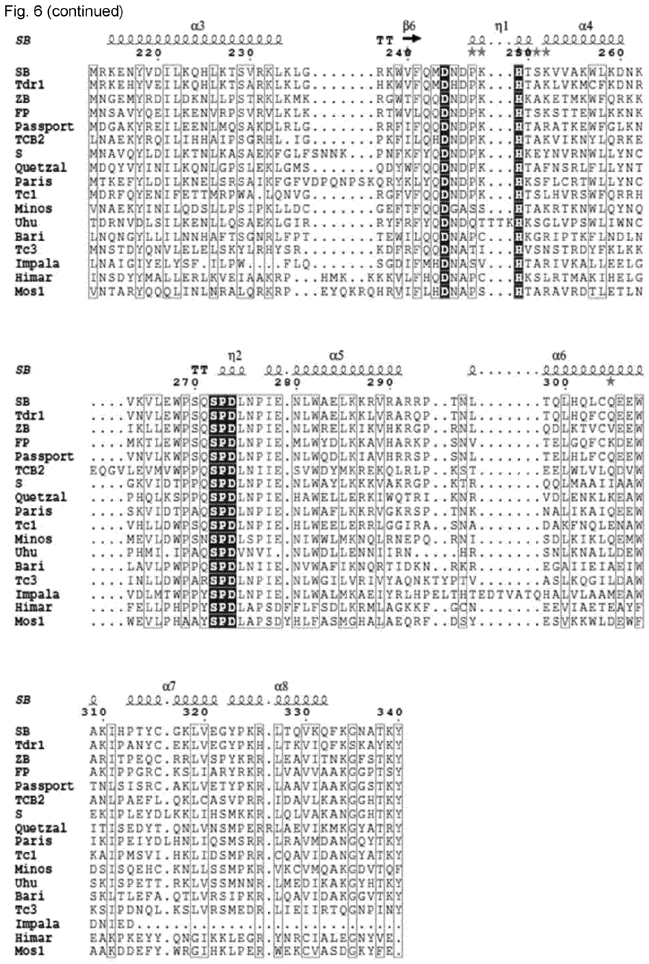

Other transposases of the Tc1/*mariner* superfamily, e.g., Frog Prince, have a similar structure, as shown in **Fig. 6****.**

Since the discovery of the SB transposon, several mutations were identified leading to an overall higher integration efficiency. These mutations culminated in the currently most active transposase variant of SB called SB100X [8]. Other mutations broadened the application of SB transposase, like the K248T mutation leading to an integration-deficient SB transposase [9], or the K248R mutation leading to a safer integration profile [10].

The WVPHEL (SEQ ID NO: 25) motif of the Tc1/*mariner* transposase Mos1 (aa 119 - 124) forming a dimer interaction interface plays an important role in downregulating the transposition reaction by an allosteric mechanism [11]. Mutations in this region were shown to lead to hyperactive *mariner* transposase variants [11].

However, this motif does not exist in all Tc1/*mariner* transposases. For example, SB, the most widely used transposase, has a KKPLL (SEQ ID NO: 26) in the positions 119-123 homologous to the WVPHEL motif that is present in Mos1 (see alignment in Fig. 6), and closely related transposases of the family, such as Frog Prince (FP), ZB, Tdr1 and Passport have similar motifs as SB.

Ivics et al. have previously demonstrated [26] that a deletion of aa 117-123, containing the ARKKPLL (SEQ ID NO: 27) motif of SB, destroys nuclear localization. Because this motif in SB aligns to the WVPHEL motif in the *mariner* transposase, the mutations that led to hyperactivity in mariner are unlikely to work in SB, because those mutations would inhibit nuclear localization.

In light of this, the inventors addressed the problem of providing further advantageous variants of transposases of the Tc1/*mariner* superfamily, e.g., having an increased transposition efficiency.

This problem is solved by the subject-matter of the claims. In particular, the invention provides a polypeptide with transpositional activity comprising or consisting of a Tc1/*mariner* superfamily transposase having a substitution in the amino acid position 124. The amino acid in position 124 is not Q.

Outside the region corresponding to the WVPHEL motif of Mos1 (aa 119 - 123), but right next to it at amino acid position 124 in the SB100X coding sequence, the inventors surprisingly identified a position that can be substituted, and in which a variant with a mutation, in particular, a substitution of glutamine 124 to cysteine 124 (Q124C), displays a -2-fold increase in transposition activity and shows resistance to the overproduction inhibition described in various Tc1/*mariner* transposases [11]. The introduction of the Q124C mutation also increases the efficiency of other, clinically relevant SB transposase variants, including K248R. Overall, these findings demonstrate utility of the mutation in position 124, in particular, the Q124C mutation, for enabling increased rates of SB transposon-mediated genome engineering in preclinical and clinical applications.

The term "transposase" as used herein refers to an enzyme that is, as a component of a functional nucleic acid-protein complex, capable of mediating a transposition reaction. A "transposition reaction" as used herein refers to a reaction where a transposon inserts into a target nucleic acid. Primary components in a transposition reaction are a transposon and a transposase or an integrase enzyme. Suitable TIR sequences of transposons are known in the art for the respective transposases. For example, pT2 or pT4 transposons, preferably, pT4 transposons can be transposed by SB.

The polypeptide with transpositional activity comprises or consists of a transposase, i.e., it can be a fusion protein of a transposase. A fused protein can be fused N-terminally or C-terminally, however, as described in more detail below, the named position is determined in relation to the transposase portion of the polypeptide only. In particular, the polypeptide may consist of the transposase.

A transposase may lack 1 to 10 N- and/or C-terminal amino acid sequences of the full length naturally occurring transposase. Preferably, it may lack a methionine at position M1, e.g., if the transposase is comprised in a fusion protein, which comprises another protein at the N-terminus of the transposase.

The term "transpositional activity" as used herein refers to the activity of a given transposase that can be assessed in a transposition reaction. "Transposition efficiency" is used synonymously. Suitable experimental set-ups are described in the experimental section herein or may use the classical binary transposition assay as described in Ivics, 1997 Cell 91: 501-510.

The transposase of the present invention is a Tc1/*mariner* superfamily transposase. Fig. 6 depicts an alignment of sequences of transposases of the Tc1/*mariner* transposase superfamily. The alignment algorithm used to generate the alignments mentioned was CLUSTAL Omega using the following settings: dealign input sequences: no, mbed-like clustering guide-tree: yes, mbed-like clustering iteration: yes, number of combined iterations: default, max guide tree iterations: default, max hmm iterations: default. Amino acid conservation is highlighted in MView. The alignment includes a secondary structure information obtained for the catalytic domain of the SB transposase (PDB entry code: 5CR4) highlighted with ESPript3.0.

The transposase of the present invention is preferably active in human cells. Suitable transposases are, e.g., Sleeping Beauty (SB), Frog Prince (FP), Minos, ZB, Tdr1 and Passport. SB is preferred.

The respective transposases are classified by their amino acid identity to the respective "wildtpye" transposase sequence in Fig. 6. That is, if the transposase has an amino acid identity of at least 80% to any of the recited sequences, and sequence identity to one of said sequences is highest, it is classified as a transposon of said class

Preferably, the transposase is a SB transposase having at least 80% amino acid identity to SEQ ID NO: 1 (SB100X), optionally, at least 90% sequence identity, at least 95% sequence identity, at least 98% sequence identity or at least 99% sequence identity. Optionally, there are only one, two or three amino acid differences to said sequence, wherein one of the differences is the substitution in position 124.

In a preferred embodiment, the transposase is SB100X comprising a substitution in position 124, wherein the amino acid in position 124 is not Q. Said sequence is provided as SEQ ID NO: 18. SB100X is a hyperactive transposase per se, but the inventors have surprisingly shown that SB100X having a Q124C substitution (SEQ ID NO: 19) still further enhanced transpositional activity. This also applies for variants of said transposase comprising one additional substitution (such as in positions 187, 247 or 248, e.g., SB100X Q124C H187V, SEQ ID NO: 20, SB100X Q124C P247R, SEQ ID NO: 21; SB100X Q124C K248R, SEQ ID NO: 22), two additional substitutions (such as in positions 187 and 247 or 187 and 248, e.g., SB100X Q124C H187V P247R or Q124C H187V K248R), three additional substitutions (such as in positions 187, 247 and 248, e.g., SB100X Q124C H187V P247R K248R) or four additional substitutions. Transposases of the invention having at least 80%, at least 85%, at least 90%, at least 95%, at least 98%, at least 99% or 100% amino acid sequence identity to SEQ ID NO:18 are therefore preferred transposases of the invention. Preferred additional substitutions in SB are e.g., disclosed in PCT/EP2022/075007. Exemplary SB transposase variants of the invention are
a) SB100X Q124X₁, wherein X₁ is not Q, wherein X₁ preferably is C, H187X₂, wherein X₂ is not H, wherein X₂ optionally is V;
b) SB100X Q124X₁, wherein X₁ is not Q, wherein X₁ preferably is C, P247X₂, wherein X₂ is not P, wherein X₂ optionally is R;
c) SB100X Q124X₁, wherein X₁ is not Q, wherein X₁ preferably is C, K248X₂, wherein X₂ is not K, wherein X₂ optionally is R.

Alternatively, the transposase can be a Tdr1 transposase having at least 80% amino acid identity to SEQ ID NO: 2, optionally, at least 90% sequence identity, at least 95% sequence identity, at least 98% sequence identity or at least 99% sequence identity. Optionally, there are only one, two or three amino acid differences to said sequence, wherein one of the differences is the substitution in position 124.

The transposase may also be a ZB transposase having at least 80% amino acid identity to SEQ ID NO: 3, optionally, at least 90% sequence identity, at least 95% sequence identity, at least 98% sequence identity or at least 99% sequence identity. Optionally, there are only one, two or three amino acid differences to said sequence, wherein one of the differences is the substitution in position 124.

In one embodiment, the transposase can be a FP (Frog Prince) transposase having at least 80% amino acid identity to SEQ ID NO: 4, optionally, at least 90% sequence identity, at least 95% sequence identity, at least 98% sequence identity or at least 99% sequence identity. Optionally, there are only one, two or three amino acid differences to said sequence, wherein one of the differences is the substitution in position 124.

The transposase can also be a Passport transposase having at least 80% amino acid identity to SEQ ID NO: 5, optionally, at least 90% sequence identity, at least 95% sequence identity, at least 98% sequence identity or at least 99% sequence identity. Optionally, there are only one, two or three amino acid differences to said sequence, wherein one of the differences is the substitution in position 124.

Alternatively, the transposase can be a Minos transposase having at least 80% amino acid identity to SEQ ID NO: 11, optionally, at least 90% sequence identity, at least 95% sequence identity, at least 98% sequence identity or at least 99% sequence identity. Optionally, there are only one, two or three amino acid differences to said sequence, wherein one of the differences is the substitution in position 124.

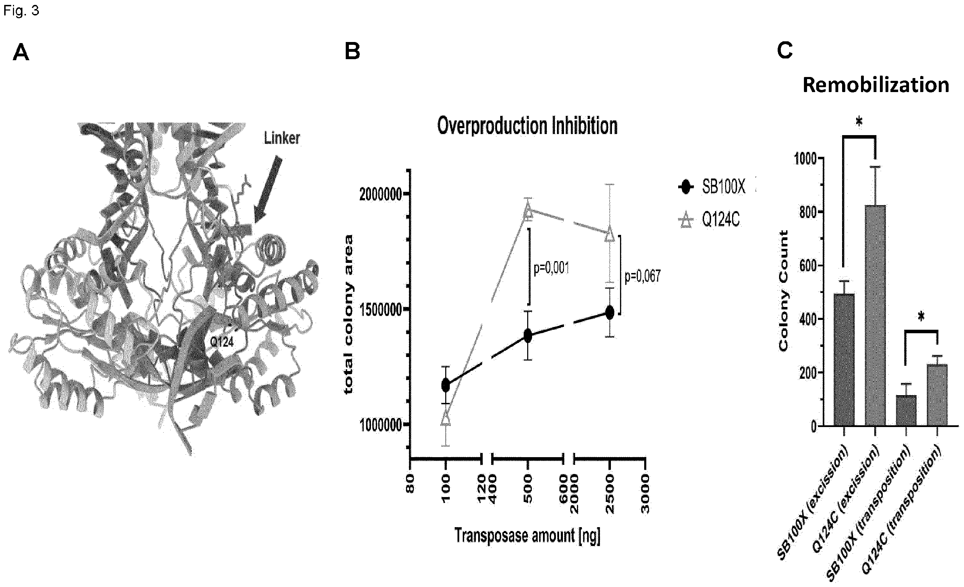

In the context of the present invention, the polypeptide of the present invention has a transpositional activity that is enhanced (i.e., more than 100%, preferably, more than 110%, more than 120%, more than 150% or about 200% or more) compared to a polypeptide that is otherwise identical, but does not comprise said substitution in position 124. A transposase not comprising said substitution has, in position 124, the amino acid residue in said position in the respective original or "wildtype" transposase as specified in the alignment of Fig. 6. For example, for a SB or Tdr1 transposase, the "wildtype" position 124 is Q. For ZB, it is N, for FP it is S and for Passport it is K. The enhanced transpositional activity does not have to be present at all transposase concentrations, but at least one concentration which is equal to or higher than the concentration at which overexpression inhibition is detected. The enhanced activity is preferably determined with an assay as described for **Fig. 3B** below.

The "position" in a transposase as used in the context of the present invention refers to the amino acid that aligns in an alignment of amino acid sequences with an amino acid sequence of a "wildtype" full length reference transposase, preferably with the full-length amino acid sequence of SB according to SEQ ID NO: 1. The position of the reference transposase is determined from the first N-terminal amino acid. Accordingly, position 124 of SB within the full-length amino acid sequence according to SEQ ID NO: 1 is "Q". Thus, an amino acid position 124 in another transposase is an amino acid that aligns with "Q" of SB at position 124 of SEQ ID NO: 1 in an alignment as shown in Fig. 6.

The amino acid in position 124 may be selected from the group consisting of C, A, R, N, D, E, G, H, I, L, K, M, F, P, S, T, W, Y and V. In one embodiment, the amino acid in position 124 is C, A, R, D, E, G, H, I, L, M, F, P, S, T, W, Y and V. The amino acid in position 124 may also be, e.g., C, A, D, E, G, H, I, L, M, F, P, W, Y and V.

If the transposase, as preferred in the context of the invention, is a SB transposase, the amino acid in position 124 can be C, A, R, N, D, E, G, H, I, L, K, M, F, P, S, T, W, Y and V. Preferred amino acids in said context are C, S or Met, most preferably, C. the same applies for a Tdr1 transposase.

If the transposase is a ZB transposase, the amino acid in position 124 can be C, A, R, D, E, G, H, I, L, K, M, F, P, S, T, W, Y and V. Preferred amino acids in said context are C, S or Met, most preferably, C.

If the transposase is a FP transposase, the amino acid in position 124 can be C, A, R, N, D, E, G, H, I, L, K, M, F, P, T, W, Y and V. Preferred amino acids in said context are C or Met, most preferably, C.

If the transposase is a Passport transposase, the amino acid in position 124 can be C, A, R, N, D, E, G, H, I, L, M, F, P, S, T, W, Y and V. Preferred amino acids in said context are C or Met, most preferably, C.

If the transposase is a Minos transposase, the amino acid in position 124 can be C, A, R, N, D, E, G, H, I, L, K, M, F, P, S, W, Y and V. Preferred amino acids in said context are C or Met, most preferably, C.

As shown herein, a significant increase in transpositional activity was achieved if the amino acid in position 124 is C. A substitution leading to C in position 124 is therefore advantageous. Accordingly, a polypeptide consisting or of comprising a SB transposase having a Q124C substitution, wherein, preferably, the transposase comprises an amino acid sequence of SEQ ID NO: 19 or 1, 2, 3 or 4 amino acids difference compared to SEQ ID NO: 19 is a preferred embodiment of the invention.

As described in PCT/EP2022/075007, transposases of the Tc1/*mariner* superfamily comprising a substitution in any of positions 187, 247 or 248, e.g., a K248R substitution, have an improved integration pattern compared to transposases not having a substitution in said positions. They have a better safety profile, as they decrease the integration of transposons into exons as well as transcriptional regulatory regions of genes in the human genome. The transposons thus integrate into genetic safe harbours with an increased frequency. It was found that these substitutions decrease transpositional activity.

However, the present inventors have shown that a substitution in position 124 can rescue transpositional activity, and lead to a more active transposase. Thus, a polypeptide of the invention further comprising a substitution in any of positions 187, 247 and/or 248 is another preferred embodiment of the invention. The polypeptide of the invention may thus also be a polypeptide as defined in PCT/EP2022/075007, in particular a polypeptide having a gain in specificity of integration into the genome. For example,
a) the amino acid in said substituted position 187 may be A, N, C, Q, G, I, L, M, S, V, W, K, R, E, P, T and S, preferably, V;
b) the amino acid in said substituted position 247 may be R, C, A or S; preferably, R, and/or
c) the amino acid in said substituted position 248 may be R, S, V, I or C, preferably, R.

For a substitution at position 187 in a SB transposase, a H187A, H187N, H187C, H187Q, H187G, H187I, H187L, H187M, H187S, H187V, H187W, H187K, H187R, H187E, H187P, H187T and H187S substitution is preferred, most preferably, a H187V substitution.

For a substitution at position 247 in a SB transposase, a P247R, P247C, P247A and P247S substitution is preferred, most preferably, a P247R substitution.

For a substitution at position 248 in a SB transposase, a K248R, K248S, K248V, K248I or K248C substitution is preferred, most preferably, a K248R substitution.

The transposase of the invention into which the substitution at position 124 is introduced can be a hyperactive transposase. In a preferred embodiment, the polypeptide according to the invention comprises SEQ ID NO: 1 or variants thereof with transpositional activity and at least 80%, 85%, 90%, 95%, 96%, 97%, 98%, 99% or 100% sequence identity to SEQ ID NO: 1, wherein the SEQ ID NO: 1 or the variant thereof comprises a substitution that increases its transpositional activity, wherein said substitution preferably is one or more of the substitutions described below. An increase in transpositional activity may be an increase compared to activity of SB of SEQ ID NO: 1. As noted above, various variants of naturally occurring transposases have been described. For example, WO 2009/003671 describes variants of transposases, in particular of SB, that have are hyperactive, i.e. that have an increased transpositional activity in comparison to the wild-type transposase, in particular SB. It is preferred that the substitutions of the invention are introduced into variants of the transposase that comprise substitutions or groups of substitutions that enhance one or more property, in particular the transpositional activity of the transposase. Thus, in one embodiment, the polypeptide according to the invention further comprises at least one of the following substitutions or groups of substitutions:
(1) K14R, K13D, K13A, K30R, K33A, T83A, I100L, R115H, R143L, R147E, A205K/H207V/K208R/D210E, H207V/K208R/D210E, R214D/K215A/E216V/N217Q; M243Q, E267D, T314N, and/or G317E;
(2) K14R//R214D/K215A/E216V/N217Q;
(3) K33A/R115H/R214D/K215A/E216V/N217Q/M243H;
(4) K14R/K30R/A205K/H207V/K208R/D210E/R214D/K215A/E216V/N217Q/M243H;
(5) K13D/K33A/T83N/H207V/K208R/D210E/M243Q;
(6) K13A/K33A/R214D/K215A/E216V/N217Q;
(7) K33A/T83N/R214D/K215NE216V/N217Q//G317E;
(8) K14R/T83A/M243Q;
(9) K14R/T83A/I100L/M243Q;
(10) K14R/T83A/R143L/M243Q;
(11) K14R/T83A/R147E/M243Q;
(12) K14R/T83A/M243Q/E267D;
(13) K14R/T83A/M243Q/T314N;
(14) K14R/K30R/I110L/A205K/H207V/K208R/D210E/R214D/K215A/E216V/N217Q/M243H;
(15) K14R/K30R/R143L/A205K/H207V/K208R/D210E/R214D/K215A/ E216V/N217Q//M243H;
(16) K14R/K30R/R147E/A205K/H207V/K208R/D210E/R214D/K215A/ E216V/N217Q/M243H;
(17) K14R/K30R/A205K/H207V/K208R/D210E/R214D/K215A/ E216V/N217Q//M243H/E267D;
(18) K14R/K30R/A205K/H207V/K208R/D210E/R214D/K215A/ E216V/N217Q/M243H/T314N;
(19) K14R/K30R/A205K/H207V/K208R/D210E/R214D/K215A/ E216V/N217Q//M243H/G317E;
(20) K14R/K33A/R115H/R214D/K215A/E216V/N217Q/M243H;
(21) K14R/K30R/R147E/A205K/H207V/K208R/D210E/R214D/K215A/ E216V/N217Q//M243H/T314N;
(22) K14R/K30R/R143U/A205K/H207V/K208R/D210E/R214D/K215A/ E216V/N217Q//M243H/E267D;
(23) K14R/K30R/R143L/A205K/H207V/K208R/D210E/R214D/K215A/ E216V/N217Q//M243H/T314N;
(24) K14R/K30R/R143L/A205K/H207V/K208R/D210E/R214D/K215A/ E216V/N217Q//M243H/G317E;
(25) K14R/K33A/R115H/R143L/R214D/K215A/E216V/N217Q/M243H;
(26) K14R/K33A/R115H/R147E//R214D/K215A/E216V/N217Q/M243H;
(27) K14R/K33A/R115H/R214D/K215A/E216V/N217Q//M243H/E267D;
(28) K14R/K33A/R115H/R214D/K215A/E216V/N217Q//M243H/T314N;
(29) K14R/K33A/R115H/R214D/K215A/E216V/N217Q//M243H/G317E;
(30) K14R/T83A/M243Q/G317E; or
(31) K13A/K33A/T83N/R214D/K215A/E216V/N217Q.

For example, the transposase of the invention may be a SB100X transposase of SEQ ID NO: 18 or, preferably, SEQ ID NO: 19.

The transposase of the invention may also be a transposase having an improved solubility in water compared to SB of SEQ ID NO: 1, e.g., a high solubility transposase as described by Querques et al. [19] or in EP3673053A. To improve solubility, the transposase may e.g. further comprise at least one substitution in the amino acid position 176 and/or 212, wherein the amino acid in position 176 is not C and the amino acid in position 212 is not I, preferably, wherein the amino acid in position 176 and/or 212 is S, wherein, optionally, both the amino acids in position 176 and 212 are S.

Another object of the invention is a nucleic acid encoding a polypeptide of the invention.

The nucleic acid of the invention may be any nucleic acid, e.g., a ribonucleic acid, including mRNA, DNA, cDNA, chromosomal DNA, extrachromosomal DNA, plasmid DNA, viral DNA, including also a recombinant viral vector. All nucleic acid variants coding for the above-mentioned inventive polypeptides are provided, including nucleic acid variants with varying nucleotide sequences due to the degeneration of the genetic code. In particular nucleotide sequences of nucleic acid variants which lead to an improved expression of the encoded fusion protein in a selected host organism, are preferred, e.g., in a human cell. Tables for appropriately adjusting a nucleic acid sequence to the host cell's specific transcription/translation machinery are known to a skilled person. In general, it is preferred to adapt the G/C-content of the nucleotide sequence to the specific host cell conditions. For expression in human cells, an increase of the G/C content by at least 10%, more preferred at least 20%, 30%, 50%, 70% and even more preferred 90% of the maximum G/C content (coding for the respective inventive peptide variant) is preferred. Preparation and purification of such nucleic acids and/or derivatives are usually carried out by standard procedures.

Preferably, the nucleic acid is suitable for expression of the polypeptide of the invention in a mammalian, e.g., human cell such as a stem cell or a lymphocyte, e.g., a T lymphocyte. If the nucleic acid is DNA, it preferably comprises at least a regulatory region of a gene. The regulatory region may be a transcriptional regulatory region, e.g., selected from the group consisting of a promoter, an enhancer, a silencer, a locus control region, and a border element. Promoters or other expression control regions can be operably linked with the nucleic acid encoding the inventive polypeptide, e.g., to regulate expression of the polypeptide/protein in a quantitative or in a tissue-specific manner. The promotor can be a constitutive or inducible promotor.

The inventive nucleic acid encoding the inventive polypeptide can be a linear fragment or a circularized, isolated fragment or be inserted into a vector, preferably as a plasmid or as recombinant viral DNA. The terms "vector" or "expression vector" are used interchangeably and refer to a polynucleotide or a mixture of a polynucleotide and proteins capable of being introduced or of introducing the nucleic acids of the present invention into a cell, preferably a mammalian cell. Examples of vectors include but are not limited to minicircles, plasmids, cosmids, phages, viruses or artificial chromosomes. In particular, a vector may be used to transport the nucleic acids of the invention into a suitable host cell. Once in the host cell, the expression vector may replicate independently of or coincidental with the host chromosomal DNA, and several copies of the vector and its inserted DNA can be generated. In case that replication incompetent expression vectors are used - which is often the case for safety reasons - the vector may not replicate but merely direct expression of the nucleic acid. Depending on the type of expression vector, the expression vector may be lost from the cell, i.e. only transiently expresses the neo-antigens encoded by the nucleic acid. It may also be stable in the cell. Expression vectors typically contain expression cassettes, i.e. the necessary elements that permit transcription of the nucleic acid into an mRNA molecule.

Preferably, the nucleic acid is mRNA, a minicircle or a plasmid. It has been shown that it is beneficial to introduce transposases into mammalian cells, in particular, stem cells or primary cells such as primary T cells, using the least amount of DNA possible, i.e., most preferably, in the form of mRNA or, alternatively, as a minicircle. If the nucleic acid is not mRNA, said nucleic acid sequence is operably linked to at least one transcription control unit, preferably, a promotor active in a human cell.

Another object of the invention is a cell comprising the nucleic acid of the invention and/or the polypeptide of the invention. Typically, the cell will comprise both. The cell may be a bacterial cell, e.g., in the context of plasmid propagation, but preferably it is a eukaryotic cell, in particular a mammalian cell. It may be, e.g., a human cell, a mouse cell, a rabbit or a rat cell. Human cells are preferred throughout the invention. The cell may be a stem cell, e.g., a pluripotent stem cell, a hematopoietic stem cell or a lymphocyte, e.g., a T lymphocyte. For example, the cell preferably is a human cell selected from the group comprising a pluripotent stem cell or a human lymphocyte, preferably, a human T lymphocyte. The cell can also be a tumor cell.

Further, the invention provides a kit comprising
a) the polypeptide of any of the invention, the nucleic acid of the invention and/or the cell of the invention; and
b) a nucleic acid comprising a cargo nucleic acid flanked by terminal inverted repeats (TIR) of a transposon capable of being mobilized by said transposase.

Such a kit may be used for delivering the cargo nucleic acid, e.g., a therapeutic amino acid, into the genome of a cell, e.g., a human cell. The cargo nucleic acid typically is an exogenous nucleic acid, i.e., a nucleic acid exogenous to the cell to which it is to be delivered. A therapeutic nucleic acid may e.g., encode a protein reduced or defective in a metabolic disease, e.g., in the context of gene therapy of said disease, a tumor suppressor gene, an immunomodulator, e.g., a cytokine, an antigen, an antibody, a T cell receptor (TCR) or chimeric antigen receptor (CAR). In a preferred embodiment, the therapeutic nucleic acid is useful for therapy of a cancer. The kit may be used for in vivo, ex vivo or in vitro applications, e.g., for use in medicine such as in cancer treatment. It may thus be a pharmaceutical kit. The cargo nucleic acid may alternatively encode a fluorescent protein and/or another selectable marker.

The invention also provides a pharmaceutical composition comprising the polypeptide of the invention, the nucleic acid of the invention; the cell of the invention, and/or the components of the kit of the invention. The pharmaceutical composition typically further comprises a pharmaceutically acceptable carrier and/or excipient. The term "carrier" refers to an organic or inorganic component, of a natural or synthetic nature, in which the active component is combined in order to facilitate, enhance or enable application. According to the invention, the term "carrier" also includes one or more compatible solid or liquid fillers, diluents, excipients or encapsulating substances, which are suitable for administration to a subject. Possible carrier substances (e.g., diluents) are, for example, sterile water, Ringer's solution, Lactated Ringer's solution, physiological saline, bacteriostatic saline (e.g., saline containing 0.9% benzyl alcohol), phosphate-buffered saline (PBS), Hank's solution, fixed oils, polyalkylene glycols, hydrogenated naphthalenes and biocompatible lactide polymers, lactide/glycolide copolymers or polyoxyethylene/polyoxy-propylene copolymers. In one embodiment, the carrier is PBS. The resulting solutions or suspensions are preferably isotonic to the blood of the recipient. Suitable carriers and their formulations are described in greater detail in Remington's Pharmaceutical Sciences, 17th ed., 1985, Mack Publishing Co.

The pharmaceutical composition preferably is for use in adoptive T cell therapy or gene therapy. Gene therapy may be therapy of a metabolic or immunological disease. In one embodiment the gene therapy includes but is not limited to autologous or heterologous T cell therapy, gene therapy targeting any cell type in the blood, hematopoietic stem cell therapy, liver gene therapy, gene therapy of the central nervous system, eye gene therapy, muscle gene therapy, skin gene therapy and/or a gene therapy for treatment of cancer.

The pharmaceutical composition of the invention may also be of interest for vaccination therapy for the integration of antigens into antigen presenting cells, e.g., human professional antigen presenting cells such as dendritic cells, macrophages or B cells or their precursors, e.g. specific tumor antigens, e.g. MAGE-1, for tumor vaccination or pathological antigens for the treatment of infectious diseases derived from pathogens, e.g. leprosy, tetanus, Whooping Cough, Typhoid Fever, Paratyphoid Fever, Cholera, Plague, Tuberculosis, Meningitis, Bacterial Pneumonia, Anthrax, Botulism, Bacterial Dysentry, Diarrhoea, Food Poisoning, Syphilis, Gasteroenteritis, Trench Fever, Influenza, Scarlet Fever, Diphtheria, Gonorrhoea, Toxic Shock Syndrome, Lyme Disease, Typhus Fever, Listeriosis, Peptic Ulcers, and Legionnaires' Disease; for the treatment of viral infections resulting in e.g. Acquired Immunodeficiency Syndrome, Adenoviridae Infections, Alphavirus Infections, Arbovirus Infections, Borne Disease, Bunyaviridae infections, Caliciviridae Infections, Chickenpox, Condyloma Acuminata, Coronaviridae Infections, Coxsackievirus Infections, Cytomegalovirus Infections, Dengue, DNA Virus Infections, Ecthyma, Contagious, Encephalitis, Arbovirus, Epstein-Barr Virus Infections, Erythema Infectiosum, Hantavirus Infections, Hemorrhagic Fevers, Viral, Hepatitis, Viral, Human, Herpes Simplex, Herpes Zoster, Herpes Zoster Oticus, Herpesviridae Infections, Infectious Mononucleosis, Influenza in Birds, Influenza, Human, Lassa Fever, Measles, Molluscum Contagiosum, Mumps, Paramyxoviridae Infections, Phlebotomus Fever, Polyomavirus Infections, Rabies, Respiratory Syncytial Virus Infections, Rift Valley Fever, RNA Virus Infections, Rubella, Slow Virus Diseases, Smallpox, Subacute Sclerosing Panencephalitis, Tumor Virus Infections, Warts, West Nile Fever, Virus Diseases, Yellow Fever; for the treatment of protozoological infections resulting in e.g. malaria. The subject methods may be used to deliver a wide variety of therapeutic nucleic acids.

According to the invention, a pharmaceutical composition contains an effective amount of the active agents, e.g., the polypeptide, nucleic acid, vector, or cell described herein, to generate the desired reaction or the desired effect. A pharmaceutical composition in accordance with the present invention is preferably sterile. Pharmaceutical compositions can be provided in a uniform dosage form and may be prepared in a manner known per se. A pharmaceutical composition in accordance with the present invention may, e.g., be in the form of a solution or suspension.

Pharmaceutically acceptable carriers, adjuvants or vehicles that may be used in the compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. The pharmaceutical compositions of the present invention may be administered orally, parenterally, by inhalation spray, topically, rectally, nasally, buccally, vaginally or via an implanted reservoir. The term parenteral as used herein includes subcutaneous, intravenous, intramuscular, intra-articular, intra-synovial, intrasternal, intrathecal, intrahepatic, intralesional and intracranial injection or infusion techniques. Preferably, the pharmaceutical compositions are administered orally, intraperitoneally or intravenously. Sterile injectable forms of the pharmaceutical compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

The inventive pharmaceutical composition is preferably suitable for the treatment of a disease, in particular a disease caused by a gene defect such as cystic fibrosis, hypercholesterolemia, hemophilia, e.g. A, B, C or XIII, immune deficiencies including HIV, Huntington disease, α-anti-Trypsin deficiency, as well as cancer selected from colon cancer, melanomas, kidney cancer, lymphoma, acute myeloid leukemia (AML), acute lymphoid leukemia (ALL), chronic myeloid leukemia (CML), chronic lymphocytic leukemia (CLL), gastrointestinal tumors, lung cancer, gliomas, thyroid cancer, mamma carcinomas, prostate tumors, hepatomas, diverse virus-induced tumors such as e.g. papilloma virus induced carcinomas (e.g. cervix carcinoma), adeno carcinomas, herpes virus induced tumors (e.g. Burkitt's lymphoma, EBV induced B cell lymphoma), Hepatitis B induced tumors (Hepato cell carcinomas), HTLV-1 und HTLV-2 induced lymphoma, akustikus neurinoma, lung cancer, pharyngeal cancer, anal carcinoma, glioblastoma, lymphoma, rectum carcinoma, astrocytoma, brain tumors, stomach cancer, retinoblastoma, basalioma, brain metastases, medullo blastoma, vaginal cancer, pancreatic cancer, testis cancer, melanoma, bladder cancer, Hodgkin syndrome, meningeoma, Schneeberger's disease, bronchial carcinoma, pituitary cancer, mycosis fungoides, gullet cancer, breast cancer, neurinoma, spinalioma, Burkitt's lymphoma, lyryngeal cancer, thymoma, corpus carcinoma, bone cancer, non-Hodgkin lymphoma, urethra cancer, CUP-syndrome, oligodendroglioma, vulva cancer, intestinal cancer, oesphagus carcinoma, small intestine tumors, craniopharyngeoma, ovarial carcinoma, ovarian cancer, liver cancer, leukemia, or cancers of the skin or the eye; etc..

The pharmaceutical composition preferably is for treatment of a human subject. The invention also provides a method of treating a subject, e.g., a human subject in need thereof, e.g., for any of the diseases mentioned herein, comprising administering an effective amount of the pharmaceutical composition of the invention or the components of the pharmaceutical kit of the invention to said subject.

Also described is the use, e.g., an in vitro use, of the polypeptide of the invention, the nucleic acid of the invention, the cell of the invention or the kit of the invention for introducing an exogenous nucleic acid into the genome of a cell.

Further, the invention provides a method, optionally, an in vitro method, of preparing a cell with an exogenous nucleic acid integrated into the genome of the cell, comprising steps of
a) providing an isolated cell;
b) providing to the cell a polypeptide of any of the invention; and
c) providing to the cell a nucleic acid comprising the exogenous nucleic acid flanked by terminal inverted repeats (TIR) of a transposon capable of being mobilized by said transposase.

Optionally, the polypeptide is provided to the cell by introducing the nucleic acid of the invention into the cell. Alternatively, the polypeptide can be provided in polypeptide form, e.g., as a high solubility transposase as described herein. In one embodiment the exogenous nucleic acid is comprised in the nucleic acid or the vector of the invention. It can also be administered separately.

In one embodiment, the nucleic acid of the invention and/or the exogenous nucleic acid is provided in the cell using a method selected from the group consisting of: electroporation, microinjection, lipoprotein particles, virus-like particles. Electroporation has been found particularly suitable for transfecting primary cells, e.g., T cells.

Another object of the invention is a method of preparing the polypeptide of the invention, comprising cultivating a cell of the invention comprising the nucleic acid of the invention and isolating the polypeptide.

The invention is further illustrated in the appended figures and examples. These are not intended to limit the invention. Cited literature is herewith fully incorporated herein by reference.

**Fig. 1****. Schematic overview of gene delivery with *Sleeping Beauty* transposition.** The SB transposase is introduced into a cell in form of DNA (e.g., an expression plasmid), mRNA or recombinant protein along with donor DNA in which the transposon to be mobilized is located. Donor DNA can be vectorized as plasmids or minicircles. After binding within the terminal inverted repeats of the transposon (TIRs, rectangles) flanking a gene of interest (GOI, rectangle), SB transposase (circles) performs the excision of the transposon from the donor DNA (black strand) and integrates it into a site in the genomic target DNA (strand).

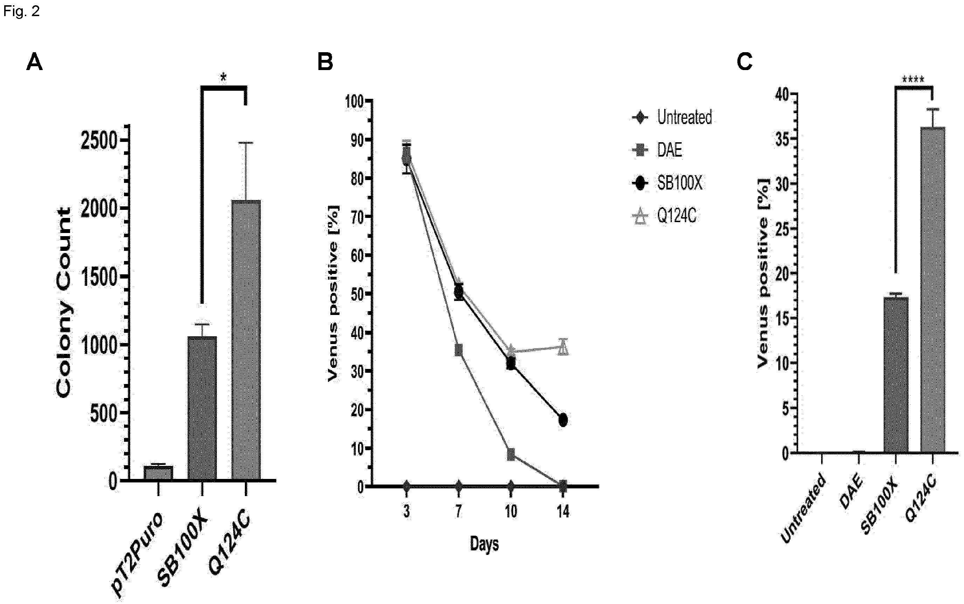

**Fig. 2****. Validation of Q124C mutation in the *Sleeping* Beauty transposase.** (A) The Q124C mutant of SB100X displays a hyperactive phenotype, as assayed by a colony-forming transposition assay in human HepG2 cells. **(B)** Venus expression in SB-transfected hiPSCs, measured by flow cytometry, reached stable levels at day 14. **(C)** The Q124C mutant leads to increased rates of stably Venus-modified hiPSCs, measured by flow cytometry.

**Fig. 3****. Mechanistic insights for hyperactivity of the Q124C mutation in the *Sleeping Beauty* transposase. (A)** Model of the SB transposase and the Q124 aa side chain (highlighted) bound to the SB transposon and target DNA. **(B)** The Q124C mutant displays its hyperactivity especially at high doses, assays by a dose dependent colony-forming transposition assay in human HepG2 cell. **(C)** In a remobilization colony forming assay with puro-selection (excision) and G418 & puro-double selection (transposition) the Q124C mutant has an increased excision rate, leading to overall higher transposition rate compared to SB100X.

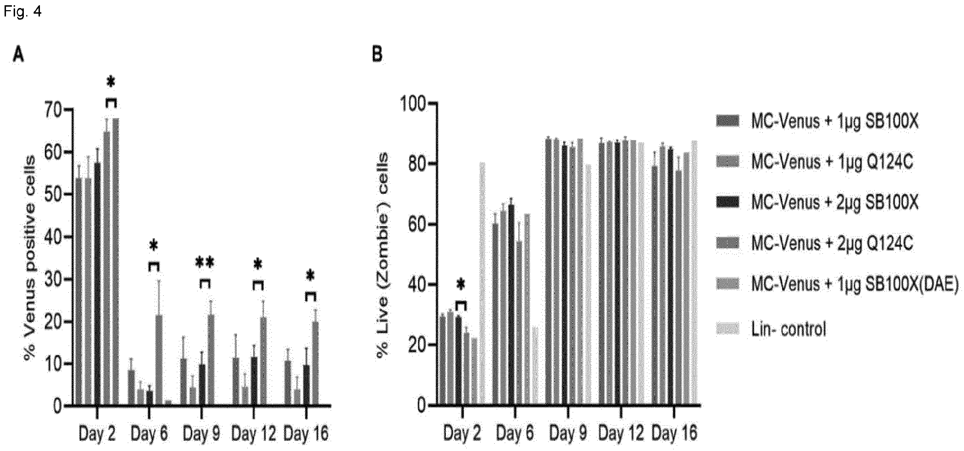

**Fig. 4****. Validation of the Q124C mutation in mouse primary hematopoietic stem cells (HSCs). (A)** The Q124C mutant of SB100X displays a hyperactive phenotype in HSCs at high doses, as assayed by Venus expression in SB nucleofected HSCs. At day 9 Venus expression reaches stable expression levels. **(B)** Viability of nucleofected HSCs with minicircle Venus and SB transposase, assayed by Zombie-staining and flow-cytometry analysis over time.

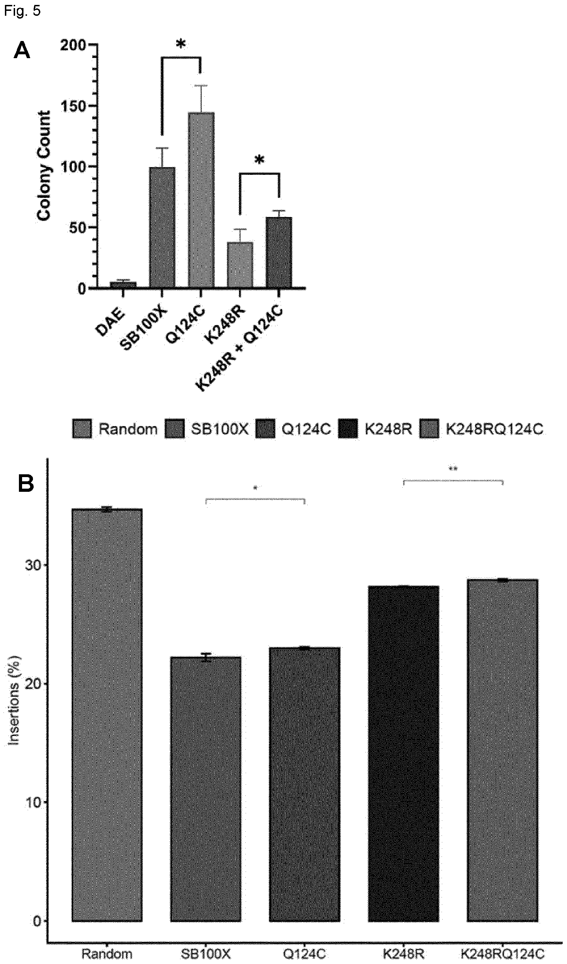

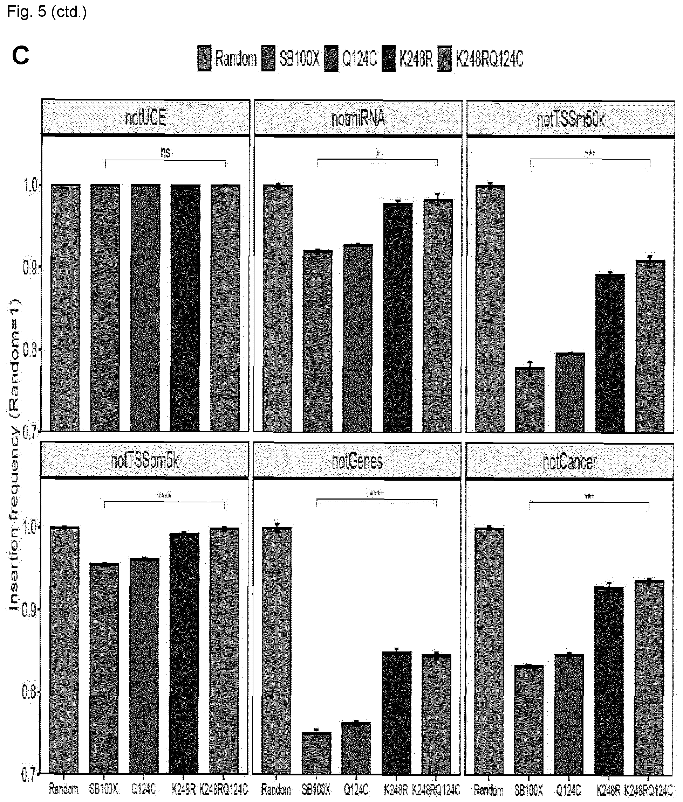

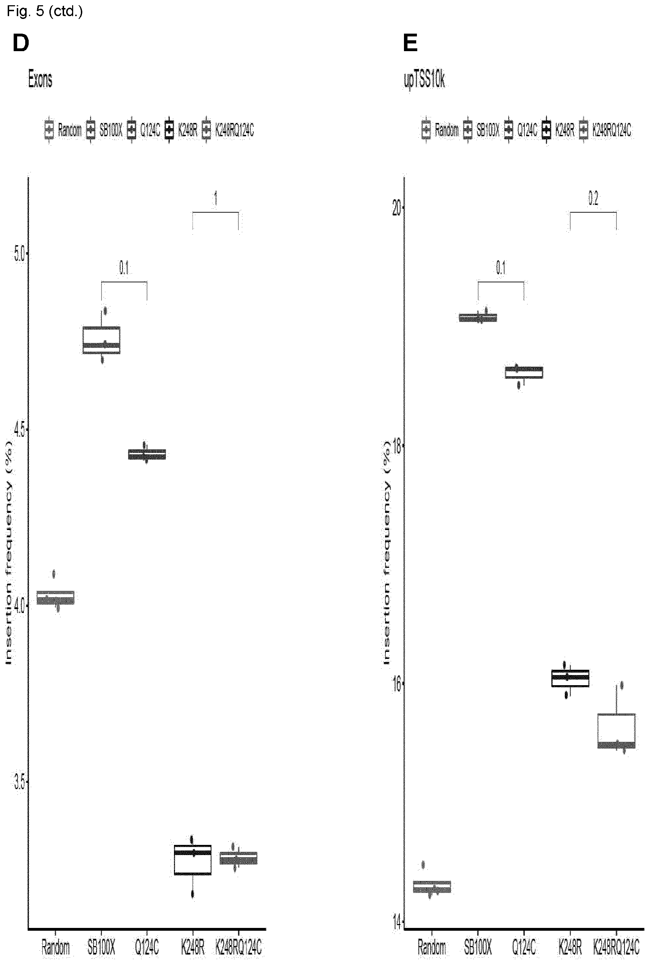

**Fig. 5****. Combination of the Q124C mutant with other *Sleeping Beauty* transposase variants rescues transposition activity. (A)** The introduction of the Q124C mutant to the K248R mutant rescues some level of its transposition activity, as assayed by a colony-forming transposition assay in human HepG2 cells. **(B)** Insertion frequency into genomic safe harbors. **(C)** Insertion frequency into all genomic safe harbor subcategories. **(D)** Representation of insertions into exons. **(E)** Representation of insertions 10 kb upstream of transcriptional start sites (TSS).

**Fig. 6****. Alignment of the amino acid sequences of Tc1/*mariner* transposase closely related to Sleeping Beauty transposase (SEQ ID NO: 1-17).** Fig. 6 allows the skilled person to determine the amino acid position within all of the other aligned transposases that correspond to amino acid position 124 or other specified amino acid positions, which could be substituted with a different amino acid in the same way as outlined exemplary for SB. Further transposases could be added to the alignment allowing the identification of amino acids corresponding to amino acid position 124 of SB in these transposases. On top of the sequence alignment the secondary structures typical for transposases of the Tc1/*mariner* superfamily: α1 helix- α2 helix- β1 sheet- β2 sheet- β3 sheet-β4 sheet- β5 sheet-α3 helix-β6 sheet- η1-α4 helix-η2-α5 helix- α6 helix-α7 helix-α8 helix belonging to the corresponding amino acid stretches are indicated. The amino acid sequence of SB (SEQ ID NO: 1) was used as the reference amino acid sequence in this alignment. All amino acids corresponding to amino acid position 187, 247 and 248 of SB are highlighted with a box.

### Examples

### Results

### The Q124C mutation leads to hyperactivity in human hepatocyte-derived HepG2 cells and in human induced pluripotent stem cells (hiPSCs)

To assay transposition activity of the Q124C mutation in the SB100X coding sequence, we transfected expression plasmids (pcGlobin2) encoding SB100X or the Q124C mutant into HepG2 cells alongside a puromycin resistance (puro) gene-tagged SB vector (pT2/Puro) and assessed the relative efficiencies of transposition by counting puro-resistant cell colonies following antibiotic selection. The Q124C mutant leads to a two-fold increase in transposition rate in comparison to the currently most active SB transposase variant SB100X **(****Fig. 2A****).** This hyperactivity also displayed itself in more clinically relevant human induced pluripotent stem cells (hiPSCSs). hiPSCs were transfected with expression plasmids encoding for the two active SB transposase variants SB100X and Q124C and the catalytically inactive variant DAE alongside a Venus fluorescence gene-tagged SB transposon minicircle. The fluorescence of the hiPSCs was followed over time and reached at day 14 stable expression **(****Fig. 2B****).** At day 14 the Q124C variant displayed a two-fold increase in the numbers of Venus-modified hiPSCs in comparison to the SB100X variant **(****Fig. 2C****).**

### The Q124C mutation leads to resistance to overproduction inhibition and manifests itself already at the excision step

To get a better understanding about the mechanistic reasons for the hyperactivity of the Q124C mutant in the SB100X coding sequence, transposition assays were performed at different doses of SB transposase expression plasmids. It is a known phenomenon in Tc1/*mariner* TEs that high levels of transposase lead to overproduction inhibition and therefore a nonlinear saturated increase in transposition rates [11]. Since the mutation site at Q124 is close to the protein-protein dimer interaction interface prone for the allosteric implementation of the overproduction inhibition effect, we speculate that the hyperactivity of the Q124C mutant should manifest itself especially at high transposase concentrations. The position of Q124 is highlighted in a model of the SB transposase together with the SB transposon ends and the DNA target site **(****Fig. 3A****).**The model is based on the crystal structure of the catalytic domain [12] and the NMR structure of the PAI [13] and RED [14] subdomain and superposed with the full-length structure of the related *mariner* transposase Mos1 [15].

In the canonical transposition assay, leading to puro-resistant colonies, comparing SB100X with Q124C, the transposition hyperactivity is displayed at higher doses (500 & 2500 ng of expression plasmid) **(****Fig. 3B****).** These observations lead us to conclude that the Q124C variant has a certain level of resistance to overproduction inhibition. Hence, the Q124C variant expands the dose of SB transposase amount that can be used in a preclinical and clinical context, leading to overall higher transposition rates.

We have established a transgenic, human, HepG2-derived reporter cell line that harbors a single copy of a neomycin resistance gene (neo)-tagged SB transposon that disrupts the open reading frame (ORF) of a puro-resistance gene. These cells are thus G418-resistant and puro-sensitive. If the SB transposase is expressed in these reporter cells, canonical excision of the SB transposon and subsequent repair of the broken DNA ends by the non-homologous end joining (NHEJ) double-strand DNA repair pathway reconstitute the puro ORF, thereby resulting in a selectable, puro-resistant phenotype. By selection with puro the excision rate of different transposase variants can be measured, while double selection with G418 and puro enables scoring of the efficiency of the full transposition reaction (excision + integration). Transfecting this reporter cell line with the two variants SB100X and Q124C shows that hyperactivity manifests itself already at the excision step, leading to an overall increased transposition rate **(****Fig. 3C****).** This goes in line with the described effect of resistance to overproduction inhibition, since this effect is mainly influencing the initiation of the excision complex.

### The Q124C mutation leads to hyperactivity in hematopoietic stem cells (HSCs)

To assay transposition activity of the Q124C mutation in the SB100X coding sequence in primary hematopoietic stem cells (HSCs), lineage negative bone marrow HSCs were isolated from mice and nucleofected with expression plasmids encoding for the two active SB transposase variants SB100X and Q124C and the catalytically inactive variant DAE alongside a Venus fluorescence gene tagged SB minicircle. The fluorescence **(****Fig. 4A****)** and viability **(****Fig. 4B****)** of the HSCs was followed over time and reached stable expression at day 9. While at a dose of 1 µg expression plasmid no difference in Venus-positive HSCs could be observed, at a high dose of 2 µg expression plasmid Q124C mutation lead to a two fold increase in stably expressing Venus-positive HSCs **(****Fig. 4A****).** The viability of the HSCs was impacted only mildly at a high dose of Q124C and recovered quickly at day 9 (Fig. 4B). This experimental data shows that the hyperactivity of the Q124C mutant also manifests itself in primary cells.

### Introduction of the Q124C mutation to K248R mutation rescues transposition activity

Other SB transposase variants like the K248R mutation in the SB100X coding sequence offer certain advantages, like a safer integration profile in comparison to SB100X [10]. The K248R variant has a decreased rate to integrate into genes and promotor regions, and an increased rate to integrate into genomic locations considered as safe harbors. However, the transposition activity of this variant is reduced in comparison to SB100X. By introducing the Q124C mutation to the K248R version the transposition activity of this variant can be rescued to some extent. The transposition activity of the different variants has been assayed in a standard colony-forming assay, counting puro-resistant colonies **(****Fig. 5A****).**

Integration of therapeutic gene constructs mediated by SB transposition into safe sites of the human genome would prevent insertional genome toxicity and the associated risk of oncogenesis. Genomic "safe harbors" are regions of the human genome that can tolerate integrations of new DNA without adverse effects on the host cell. Chromosomal sites or regions can be bioinformatically assigned as GSHs if they satisfy the following criteria: (i) no overlap with transcription units, (ii) distance of at least 50 kb from the 5'-end of any gene, (iii) at least 300 kb distance to cancer related genes and (iv) microRNA genes, and (v) regions outside of ultra-conserved elements (UCEs) [16,17]. The insertion frequency into GSHs was increased by the introduction of the Q124C mutation into the coding sequence of the SB transposase **(****Fig. 5B****).** In all subcategories of the GSHs the double mutant Q124C/K248R had in increased insertion frequency **(****Fig. 5C****).** Assaying the representation of insertions into exons **(****Fig. 5D****)** and 10 kb upstream of transcriptional start sites **(****Fig. 5E****)** the Q124C single mutation had a lower insertions frequency compared to SB100X, while the double mutant (Q124C/K248R) preserved its lower insertion frequency in comparison to the single mutant (K248R).

The introduction of the Q124C mutant into the K248R variant lead to an increased value of this variant in clinical application, where efficiency and safety especially in comparison to viral vectors play an important role. It is visible that the Q124 residue is located more on the protein surface involved in the DNA interaction **(****Fig. 3A****).** Therefore, it is plausible that other SB transposase variants can also be combined with the Q124C mutant to increase their transposition activity.

### Material and Methods

### Site directed mutagenesis of the SB100X transposase

All mutations were generated using the Q5 polymerase (NEB, Ipswich, MA, USA) and the plasmid pcGlobin2-SB100X. 5'-phosphorylated primers for the particular positions were designed with 5'-ends annealing back-to-back. Primers were synthesized with a 5'-phosphate to enable a downstream intramolecular ligation reaction and were ordered from Eurofins (Eurofins/MWG, Luxembourg). Primer sequences (SB100X-Q124_to_C-rev: 5'Phos-CTTCTTCCTTGCTGAGTGG-3' (SEQ ID NO: 23) & SB100X-Q124_to_C124-fwd: 5'Phos-CCACTGCTCTGCAACCGACATAAGAAAGCC-3' (SEQ ID NO: 24)). PCR cycling conditions were set according to the manufacturer's instructions. The annealing temperatures of the mutagenic primers were calculated with the "NEB Tm calculator^{™}" software (https://www.neb.com/tools-and-resources/interactive-tools/tm-calculator). The PCR products were purified with QIAquick PCR Purification Kit (QIAGEN, Venlo, Holland), eluted in 30 µl elution buffer and digested with 2 µl *Dpn*I (NEB, Ipswich, MA, USA) for 2 h at 37°C, followed by 20 min heat-inactivation at 80°C. The linear, double-stranded PCR products were circularized by ligation with T4 DNA Ligase (NEB, Ipswich, MA, USA) overnight at 16 °C. The circularized PCR products were transformed into chemically competent *E*. *coli* (Invitrogen/Life Technologies, Carlsbad, CA, USA), grown in Luria-Bertani (LB) medium for 1 h, and selected for ampicillin resistance by plating on LB agar plates containing 100 µg/ml ampicillin. To confirm the presence of the desired mutations, and the absence of undesired mutations, plasmid DNA from several colonies was purified using the QIAprep spin miniprep kit (QIAGEN, Venlo, Holland) and Sanger sequenced by Eurofins (Eurofins/MWG, Luxembourg).

### Transposition Assay

For transposition assay in HepG2 cells, 3×10^5 cells were seeded onto six-well plates one day prior to transfection. Transfection was done with TransIT-LT1 transfection reagent (Mirus Bio LLC, Madison, WI, USA) following manufacturers protocol. Each transfection reaction was filled up to the same total DNA amount transfected with the plasmid pmaxGFP. The transposon expression plasmid (pcGlobin2) were co-transfected with the transposon donor plasmid (pT2/Puro). Forty-eight hours after transfection cells were trypsinized and 1-10% of the cells were replated to 10-cm plates, and selected for transposon integration using 1 µg/ml puromycin (InvivoGen, San Diago, CA, USA). After 2 weeks of selection, cell colonies were fixed with 10% (vol/vol) formaldehyde in phosphate-buffered saline (PBS), stained with methylene blue in PBS, and counted. For in vitro comparisons of relative transposition efficiencies at least three independent experiments were performed.

### Venus modification of hiPSCs with Sleeping Beauty

hiPSCs were cultured and handled following the protocol of Skarnes et al. [18]. Transfection was done with TransIT-LT1 transfection reagent (Mirus Bio LLC, Madison, WI, USA) following manufacturers protocol for hiPSCs. 2 µg of the transposon expression plasmid (pcGlobin2) were co-transfected with the transposon donor minicircle (mcVenus) to 2×10^6 hiPSCs. Venus expression were assessed every three to four days by cytofluorimetric analysis with the BD FACS-SORB (BD FACS^{™}, East Rutherford, NJ, USA) and analyzed by FlowJo (FlowJo LLC, Asheland, OR, USA). For in vitro comparisons of relative transposition efficiencies at least three independent experiments were performed.

### Remobilization Assay

For remobilization assay in the modified HepG2 reporter cell line (HepG2-PB(SB2#23)), 4×10^5 cells were seeded onto six-well plates one day prior to transfection. Transfection was done with Lipofectamine 3000 transfection reagent (Invitrogen AG, Waltham, MA, USA) following manufacturers protocol. 1 µg of the transposon expression plasmid (pcGlobin2) was transfected to the HepG2-PB(SB2#23) cells. Seventy-two hours after transfection cells were trypsinized and 100% of the cells were replated on two 10-cm plates, and either selected for transposon excision using 1 µg/ml puromycin (InvivoGen, San Diago, CA, USA), or double selected for transposition using 1 µg/ml puromycin and 1 mg/ml G418 (InvivoGen, San Diago, CA, USA). After 3 weeks of selection, cell colonies were fixed with 10% (vol/vol) formaldehyde in phosphate-buffered saline (PBS), stained with methylene blue in PBS, and counted. For in vitro comparisons of relative transposition efficiencies at least three independent experiments were performed.

### Generation of SB insertion libraries

For the analysis of the target site selection properties of the SB100X transposase mutants, 4 × 10^5 HepG2 cells were seeded onto 6-well plates 1 day prior to transfection. Transfections were done with QIAGEN-purified plasmid DNA using TransIT-LT1 transfection reagent according to the manufacturer's protocol. One µl of TransIT-LT1 transfection reagent were used to transfect 550 ng of DNA, including 500 ng pT2Bpuro, 50 ng helper plasmid expressing the mutant transposases or pcGlobin2-SB100X. 48 h after transfection, cells were trypsinized, diluted to multiple 10 cm dishes containing DMEM supplemented with 1 µg/ml puromycin and selected for growth over a period for 2 weeks. At least 10 000 puromycin-resistant HepG2 cell colonies were trypsinized and centrifuged at 1000 rpm for 5 min. The pellet was washed with PBS and genomic DNA was extracted from cells using the Qiagen DNeasy Blood & Tissue Kit according to manufacturer's protocol.

For the generation of SB insertion site libraries, 2 µg DNA was sheared with a Covaris M220 ultra-solicitor device to an average fragment size of 600 bp in Screw-Cap microTUBEs in 50 µl, using the following settings: peak incident power 50 W, duty factor 20%, cycles per burst 200, treatment 28 s. 1.2 µg of the sheared DNA was blunted and 5'-phosphorylated using the NEBNext End Repair Module (NEB), and 3'-A-tailed with NEBNext dA-Tailing Module (NEB) following the recommendations of the manufacturer. The DNA was purified with the Clean and Concentrator Kit (Zymo Research) and eluted in 8 µl 10 mM Tris pH 8.0 (EB) for ligation with 50 pmol of T-linker (see below) with T4 ligase (NEB) in 20 µl volume, at 16 °C, overnight. T-linkers were created by annealing the 100 pmol each of the oligonucleotides Linker_TruSeq_T+ and Linker_TruSeq_T- in 10 mM Tris-CI pH 8, 50 mM NaCl, 0.5 mM EDTA. After heat-inactivation, ligation products enclosing fragments of non-integrated transposon donor plasmid DNA were digested with Dpnl (NEB) in 50 µl for 3 h, and the DNA was column-purified and eluted in 20 µl EB. Six µl elute was used for PCR I with 25 pmol of the primers specific for the linker and for the transposon inverted repeat: Linker and T-Bal-Long, respectively, with the conditions: 98 °C 30 s; 10 cycles of 98 °C 10 s, 72 °C 30 s; 15 cycles of 98 °C 10 s, ramp to 62 °C (1 °C/s) 30 s, 72 °C 30 s, 72 °C 5 min. All PCR reactions were performed with NEBNext High-Fidelity 2 × PCR Master Mix. The PCR was column purified eluted in 20 µl EB and 10 µl was used for PCR II with primers Nested and LAM-SB-50, with the following program: 98 °C 30 s; 12 cycles of 98 °C 10 s, ramp to 65 °C (1 °C/sec) 30 s, 72 °C 30 s, 72 °C 5 min. One third of the column-purified PCR II was used for PCR III with primers PE-nest-ind-N and SB-20-bc-ill-N (where N is the number of the Illumina TrueSeq indexes) for barcoding the samples using the following PCR program: 98°C 30 s; 12 cycles of 98 °C 10 s, ramp to 64 °C (1 °C/s) 30 s, 72 °C 30 s, 72 °C 5 min. The final PCR products were separated on a 1% agarose gel and the smears of 200-500 bp were isolated and purified.

### Sequencing and analyses of the insertion sites

The insertion site libraries were prepared as described earlier [19] and sequenced on Illumina instruments with 150-bp, single-end settings. After adapter and quality trimming (Phred score ≥ 20) with fastp [20] the reads were tested for the transposon sequences downstream of the SB-specific primer and were filtered for the presence of the remaining part of the transposon inverted terminal repeat (ITR) and a minimum length of 28 bases of genomic sequence for mapping with bowtie2 [21] with -sensitive and -end-to-end settings. A mapped locus was considered valid if the mapping quality for the read supporting it was ≥20. Any insertion site needed to be supported by at least 10 independent reads at a TA target site of the human genome (hg38). If multiple insertions were detected within 10 bases the insertion site supported by the largest number of independent reads were consider as the valid one. Representation of the insertion sites in various genic categories were investigated using the Genomation package [22]. A computationally generated random set of 100 000 loci of the human hg38 genome assembly was used as reference for investigations of insertion site representation in various genomic bins. The genomic safe harbor coordinates for the hg38 assembly were created following the earlier defined criteria [16].

### References

1. McClintock, B. Induction of Instability at Selected Loci in Maize. Genetics 1953, 38, 579-599.
2. Wicker, T.; Sabot, F.; Hua-Van, A.; Bennetzen, J.L.; Capy, P.; Chalhoub, B.; Flavell, A.; Leroy, P.; Morgante, M.; Panaud, O.; et al. A unified classification system for eukaryotic transposable elements. Nat. Rev. Genet. 2007, 8, 973-982, doi:10.1038/nrg2165.
3. Ivics, Z.; Hackett, P.B.; Plasterk, R.H.; Izsvák, Z. Molecular Reconstruction of Sleeping Beauty, a Tc1-like Transposon from Fish, and Its Transposition in Human Cells. Cell 1997, 91, 501-510, doi:10.1016/S0092-8674(00)80436-5.
4. Amberger, M.; Ivics, Z. Latest Advances for the Sleeping Beauty Transposon System: 23 Years of Insomnia but Prettier than Ever: Refinement and Recent Innovations of the Sleeping Beauty Transposon System Enabling Novel, Nonviral Genetic Engineering Applications. Bioessays 2020, 42, e2000136, doi:10.1002/bies.202000136.
5. Izsvák, Z.; Khare, D.; Behlke, J.; Heinemann, U.; Plasterk, R.H.; Ivics, Z. Involvement of a bifunctional, paired-like DNA-binding domain and a transpositional enhancer in Sleeping Beauty transposition. J. Biol. Chem. 2002, 277, 34581-34588, doi:10.1074/jbc.M204001200.
6. Montaño, S.P.; Rice, P.A. Moving DNA around: DNA transposition and retroviral integration. Curr. Opin. Struct. Biol. 2011, 21, 370-378, doi:10.1016/j.sbi.2011.03.004.
7. Yang, W.; Lee, J.Y.; Nowotny, M. Making and breaking nucleic acids: two-Mg2+-ion catalysis and substrate specificity. Molecular Cell 2006, 22, 5-13, doi:10.1016/j.molcel.2006.03.013.
8. Mátés, L.; Chuah, M.K.L.; Belay, E.; Jerchow, B.; Manoj, N.; Acosta-Sanchez, A.; Grzela, D.P.; Schmitt, A.; Becker, K.; Matrai, J.; et al. Molecular evolution of a novel hyperactive Sleeping Beauty transposase enables robust stable gene transfer in vertebrates. Nat. Genet. 2009, 41, 753-761, doi:10.1038/ng.343.
9. Kesselring, L.; Miskey, C.; Zuliani, C.; Querques, I.; Kapitonov, V.; Laukó, A.; Fehér, A.; Palazzo, A.; Diem, T.; Lustig, J.; et al. A single amino acid switch converts the Sleeping Beauty transposase into an efficient unidirectional excisionase with utility in stem cell reprogramming. Nucleic Acids Res. 2020, 48, 316-331, doi:10.1093/nar/gkz1119.
10. Miskey, C.; Kesselring, L.; Querques, I.; Abrusán, G.; Barabas, O.; Ivics, Z. Engineered Sleeping Beauty transposase redirects transposon integration away from genes. Nucleic Acids Res. 2022, 50, 2807-2825, doi:10.1093/nar/gkac092.
11. Liu, D.; Chalmers, R. Hyperactive mariner transposons are created by mutations that disrupt allosterism and increase the rate of transposon end synapsis. Nucleic Acids Res. 2014, 42, 2637-2645, doi:10.1093/nar/gkt1218.
12. Voigt, F.; Wiedemann, L.; Zuliani, C.; Querques, I.; Sebe, A.; Mátés, L.; Izsvák, Z.; Ivics, Z.; Barabas, O. Sleeping Beauty transposase structure allows rational design of hyperactive variants for genetic engineering. Nat. Commun. 2016, 7, 11126, doi:10.1038/ncomms11126.
13. Carpentier, C.E.; Schreifels, J.M.; Aronovich, E.L.; Carlson, D.F.; Hackett, P.B.; Nesmelova, I.V. NMR structural analysis of Sleeping Beauty transposase binding to DNA. Protein Sci. 2014, 23, 23-33, doi:10.1002/pro.2386.
14. Konnova, T.A.; Singer, C.M.; Nesmelova, I.V. NMR solution structure of the RED subdomain of the Sleeping Beauty transposase. Protein Sci. 2017, 26, 1171-1181, doi:10.1002/pro.3167.
15. Richardson, J.M.; Colloms, S.D.; Finnegan, D.J.; Walkinshaw, M.D. Molecular architecture of the Mos1 paired-end complex: the structural basis of DNA transposition in a eukaryote. Cell 2009, 138, 1096-1108, doi:10.1016/j.cell.2009.07.012.
16. Sadelain, M.; Papapetrou, E.P.; Bushman, F.D. Safe harbours for the integration of new DNA in the human genome. Nat. Rev. Cancer 2011, 12, 51-58, doi:10.1038/nrc3179.
17. Papapetrou, E.P.; Lee, G.; Malani, N.; Setty, M.; Riviere, I.; Tirunagari, L.M.S.; Kadota, K.; Roth, S.L.; Giardina, P.; Viale, A.; et al. Genomic safe harbors permit high β-globin transgene expression in thalassemia induced pluripotent stem cells. Nat. Biotechnol. 2011, 29, 73-78, doi:10.1038/nbt.1717.
18. Skarnes, W.C.; Pellegrino, E.; McDonough, J.A. Improving homology-directed repair efficiency in human stem cells. Methods 2019, 164-165, 18-28, doi:10.1016/j.ymeth.2019.06.016.
19. Querques, I.; Mades, A.; Zuliani, C.; Miskey, C.; Alb, M.; Grueso, E.; Machwirth, M.; Rausch, T.; Einsele, H.; Ivics, Z.; et al. A highly soluble Sleeping Beauty transposase improves control of gene insertion. Nat. Biotechnol. 2019, 37, 1502-1512, doi:10.1038/s41587-019-0291-z.
20. Chen, S.; Zhou, Y.; Chen, Y.; Gu, J. fastp: an ultra-fast all-in-one FASTQ preprocessor. Bioinformatics 2018, 34, i884-i890, doi:10.1093/bioinformatics/bty560.
21. Langmead, B.; Salzberg, S.L. Fast gapped-read alignment with Bowtie 2. Nat. Methods 2012, 9, 357-359, doi:10.1038/nmeth.1923.
22. Akalin, A.; Franke, V.; Vlahoviček, K.; Mason, C.E.; Schübeler, D. Genomation: a toolkit to summarize, annotate and visualize genomic intervals. Bioinformatics 2015, 31, 1127-1129, doi:10.1093/bioinformatics/btu775.
23. PCT/EP2022/075007
24. WO 2009/003671
25. EP3673053A
26. Ivics, Z.; Izsvak Z; Minter A; Hackett PB. Identification of functional domains and evolution of Tc1-like transposable elements. Proc Natl Acad Sci USA. 1996, 93(10):5008-13, doi: 10.1073/pnas.93.10.5008.

## Claims

1. A polypeptide with transpositional activity comprising a Tc1/*mariner* superfamily transposase having a substitution in the amino acid position 124, wherein the amino acid in position 124 is not Q.

2. The polypeptide of claim 1, wherein the transposase is selected from the group consisting of Sleeping Beauty (SB), Frog Prince (FP), Minos, ZB and Passport.

3. The polypeptide of any of claims 1 or 2, wherein the transposase is a SB transposase having at least 80% amino acid identity to SEQ ID NO: 18.

4. The polypeptide of any of the preceding claims having an enhanced transpositional activity compared to an otherwise identical transposase not comprising said substitution in position 124.

5. The polypeptide of any of the preceding claims, wherein the amino acid in position 124 is selected from the group consisting of C, A, R, N, D, E, G, H, I, L, K, M, F, P, S, T, W, Y and V.

6. The polypeptide of any of the preceding claims, wherein the amino acid in position 124 is C.

7. The polypeptide of any of the preceding claims, wherein the transposase is a SB transposase having a Q124C substitution, wherein, preferably, the transposase comprises an amino acid sequence of SEQ ID NO: 19 or having 1, 2, 3 or 4 amino acids difference compared to SEQ ID NO: 19.

8. The polypeptide of any of the preceding claims, further comprising a substitution in any of positions 187, 247 and/or 248,
wherein, optionally,
a) the amino acid in said substituted position 187 is A, N, C, Q, G, I, L, M, S, V, W, K, R, E, P, T and S, preferably, V;
b) the amino acid in said substituted position 247 is R, C, A or S; preferably, R, and/or
c) the amino acid in said substituted position 248 is R, S, V, I or C, preferably, R.

9. The polypeptide of any of the preceding claims, further comprising at least one of the following substitutions or groups of substitutions:
(1) K14R, K13D, K13A, K30R, K33A, T83A, I100L, R115H, R143L, R147E, A205K/H207V/K208R/D210E, H207V/K208R/D210E, R214D/K215A/E216V/N217Q; M243Q, E267D, T314N, and/or G317E;
(2) K14R//R214D/K215A/E216V/N217Q;
(3) K33A/R115H/R214D/K215A/E216V/N217Q/M243H;
(4) K14R/K30R/A205K/H207V/K208R/D210E/R214D/K215A/E216V/N217Q/M243H;
(5) K13D/K33A/T83N/H207V/K208R/D210E/M243Q;
(6) K13A/K33A/R214D/K215A/E216V/N217Q;
(7) K33A/T83N/R214D/K215NE216V/N217Q//G317E;
(8) K14R/T83A/M243Q;
(9) K14R/T83A/I100L/M243Q;
(10) K14R/T83A/R143L/M243Q;
(11) K14R/T83A/R147E/M243Q;
(12) K14R/T83A/M243Q/E267D;
(13) K14R/T83A/M243Q/T314N;
(14) K14R/K30R/I110L/A205K/H207V/K208R/D210E/R214D/K215A/ E216V/N217Q/M243H;
(15) K14R/K30R/R143L/A205K/H207V/K208R/D210E/R214D/K215A/ E216V/N217Q//M243H;
(16) K14R/K30R/R147E/A205K/H207V/K208R/D210E/R214D/K215A/ E216V/N217Q/M243H;
(17) K14R/K30R/A205K/H207V/K208R/D210E/R214D/K215A/ E216V/N217Q//M243H/E267D;
(18) K14R/K30R/A205K/H207V/K208R/D210E/R214D/K215A/ E216V/N217Q/M243H/T314N;
(19) K14R/K30R/A205K/H207V/K208R/D210E/R214D/K215A/ E216V/N217Q//M243H/G317E;
(20) K14R/K33A/R115H/R214D/K215A/E216V/N217Q/M243H;
(21) K14R/K30R/R147E/A205K/H207V/K208R/D210E/R214D/K215A/ E216V/N217Q//M243H/T314N;
(22) K14R/K30R/R143U/A205K/H207V/K208R/D210E/R214D/K215A/ E216V/N217Q//M243H/E267D;
(23) K14R/K30R/R143L/A205K/H207V/K208R/D210E/R214D/K215A/ E216V/N217Q//M243H/T314N;
(24) K14R/K30R/R143L/A205K/H207V/K208R/D210E/R214D/K215A/ E216V/N217Q//M243H/G317E;
(25) K14R/K33A/R115H/R143L/R214D/K215A/E216V/N217Q/M243H;
(26) K14R/K33A/R115H/R147E//R214D/K215A/E216V/N217Q/M243H;
(27) K14R/K33A/R115H/R214D/K215A/E216V/N217Q//M243H/E267D;
(28) K14R/K33A/R115H/R214D/K215A/E216V/N217Q//M243H/T314N;
(29) K14R/K33A/R115H/R214D/K215A/E216V/N217Q//M243H/G317E;
(30) K14R/T83A/M243Q/G317E; or
(31) K13A/K33A/T83N/R214D/K215A/E216V/N217Q.

10. A nucleic acid encoding a polypeptide according to any of the preceding claims.

11. The nucleic acid of claim 10 that is mRNA, a minicircle or a plasmid, wherein, optionally, if the nucleic acid is not mRNA, said nucleic acid sequence is operably linked to at least one transcription control unit, preferably, a promotor active in a human cell.

12. A cell comprising the nucleic acid of any of claims 10-11 and/or the polypeptide of any of claims 1-9.

13. The cell of claim 12, wherein the cell is a human cell selected from the group comprising a pluripotent stem cell or a human lymphocyte, preferably, a human T lymphocyte.

14. A kit comprising
a) the polypeptide of any of claims 1-9, the nucleic acid of any of claims 10-11 and/or the cell of any of claims 12-13; and
b) a nucleic acid comprising a cargo nucleic acid flanked by terminal inverted repeats (TIR) of a transposon capable of being mobilized by said transposase.

15. A pharmaceutical composition comprising the polypeptide of any of claims 1-9, the nucleic acid of any of claims 10-11; the cell of any of claims 12-13, and/or the kit of claim 14 and, optionally, a pharmaceutically acceptable carrier and/or excipient,
wherein the pharmaceutical composition preferably is for use in adoptive T cell therapy or gene therapy.

16. Use of the polypeptide of any of claims 1-9, the nucleic acid of any of claims 10-11, the cell of any of claims 12-13 or the kit of claim 14 for introducing an exogenous nucleic acid into the genome of a cell, wherein the use optionally is an in vitro use.

17. A method, optionally, an *in vitro* method, of preparing a cell with an exogenous nucleic acid integrated into the genome of the cell, comprising steps of
a) providing an isolated cell;
b) providing to the cell a polypeptide of any of claims 1-9; and
c) providing to the cell a nucleic acid comprising the exogenous nucleic acid flanked by terminal inverted repeats (TIR) of a transposon capable of being mobilized by said transposase;
wherein, optionally, the polypeptide is provided to the cell by introducing the nucleic acid of any of claims 10-11 into the cell.
